# EUROPÄISCHE PATENTANMELDUNG

(11) **EP 4 520 356 A1**
(43) Veröffentlichungstag der Anmeldung: **12.03.2025**
(21) Anmeldenummer: 23195820.8
(22) Anmeldetag: 06.09.2023
(51) Int. Cl.: A61L 27/24, A61K 35/32, A61K 38/39, A61L 27/36, A61L 27/60, A61P 17/02

(54) **BIOKOMPOSITE UND VERFAHREN ZU IHRER HERSTELLUNG**

(71) Anmelder: Helmholtz-Zentrum hereon GmbH, 21502 Geesthacht (DE)
(72) Erfinder: Machatschek, Rainhard, 10965 Berlin (DE); Bhuvanesh, Thanga, 86415 Mering (DE); Nie, Yan, 14513 Teltow (DE); Ma, Nan, 14197 Berlin (DE)
(74) Vertreter: Uexküll & Stolberg

(57) **Zusammenfassung**

Die vorliegende Erfindung betrifft ein Verfahren zur Herstellung von orientierten kollagenbasierten Biokompositmaterialien an der Luft-Wasser-Grenzfläche einer Lösung.

## Beschreibung

### GEBIET DER ERFINDUNG

Im Allgemeinen bezieht sich die vorliegende Erfindung auf Biopolymer- und Biokomposit-Materialien und -Strukturen sowie auf Verfahren zu deren Herstellung und Verwendung. In einigen Ausführungsformen ist die vorliegende Erfindung auf orientierte kollagenbasierte Biokompositmaterialien wie synthetische Basallamina sowie auf Verfahren zu deren Herstellung gerichtet.

### HINTERGRUND DER ERFINDUNG

Die Basallamina (Basalmembran) ist eine etwa 20 nm bis 100 nm dicke Proteinschicht, die sich an den basalen Zellpol der Epithelzellen anschließt. Sie ist eine spezialisierte Extrazellulärmatrix, die die Verbindung der Epithelzellen zum fibroretikulären, bindegewebigen Teil der Basalmembran herstellt. Die natürlich vorkommende Basallamina erfüllt mehrere Funktionen: u.a. beeinflusst sie den Stoffwechsel von Zellen, die räumliche Ausrichtung von Zellen bei der Zelldifferenzierung sowie die Zellmigration.

Die Basallamina besteht aus zwei miteinander verbundenen Proteinschichten, die eine charakteristische räumliche Anordnung aufweisen: die Lamina rara externa, eine elektronendurchlässige Schicht, die Laminin enthält, und die Lamina densa, eine elektronendichte Schicht mit Kollagen als Hauptkomponente. Das Laminin bildet dabei ein geordnetes Netzwerk mit einer definierten Struktur und verbindet die Epithelzellen mit dem Kollagen.

Das Laminin hat eine kreuzförmige Struktur, wobei der lange Arm des Kreuzes aus einer alpha-Helix aus drei Strängen (alpha, beta, gamma-Stränge) besteht und die drei kurzen Arme (beta-, gamma-Ketten und alpha-Kette) aus jeweils einer Kette bestehen. Die kurzen Arme sind zu einem Netzwerk verbunden und die langen Arme stellen den Kontakt zu den Epithelzellen her. Verschiedene Isoformen von Laminin sind bekannt, die sich in der Anzahl der vorhandenen alpha-, beta- und gamma-Proteinketten unterscheiden.

Synthetische Basallamina, insbesondere deren Laminin-Beschichtung, werden für Zellkulturen verwendet, können aber auch für andere Zwecke wie Gewebereparaturmaterialien oder Implantatbeschichtungen wie Stent-, Okkluder- oder Katheterbeschichtungen verwendet werden. Synthetische Basallamina sollten bio-mimetisch sein, d.h. sie sollten die gleiche räumliche Anordnung und Schichtdicke der Laminin-Moleküle wie in natürlich vorkommenden Basallamina aufweisen.

Verfahren zur Herstellung von synthetischen Laminin-Beschichtungen auf Kollagenschichten, welche als Grundlage für synthetische Basallamina verwendet werden können, sind bekannt. Die zwei gängigen Verfahren zur Herstellung der Beschichtung sind die Adsorption von Laminin aus einer Lösung und die Beschichtung mit Polylaminin. Bei der Adsorption aus einer Lösung wird das zu beschichtende Material in eine Laminin-Lösung eingebracht und nach einer gewissen Wartezeit aus diesem entfernt. Bei dem Polylaminin-Verfahren wird zusätzlich Säure zugegeben, wodurch sich das Laminin ähnlich wie im Körper vernetzt. Beide Verfahren sind aber nicht geeignet, eine Orientierung des Laminins wie in der Basallamina zu erreichen. Weiterhin hängt die finale Schichtdicke von einer Vielzahl von Parametern ab, insbesondere der Interaktion zwischen dem zu beschichtenden Material und Laminin, und muss daher in der Regel empirisch bestimmt werden. Zudem sind diese Methoden ineffizient, da nur ein geringer Teil des eingesetzten Laminins auf das zu beschichtende Material aufgebracht wird.

Gemäß einem weiteren Verfahren zur Erzeugung solcher Laminin-Beschichtungen werden Zellen auf dem Proteingemisch kultiviert, die die Basallamina herstellen, und danach die Zellen abgelöst. Dieses Verfahren hat den Vorteil, dass die erzeugte Beschichtung dem natürlichen Vorbild entspricht. Dieses Verfahren ist aber sehr zeitaufwändig und kostenintensiv.

Ein weiteres Verfahren, um lamininhaltige Beschichtungen für die Kultur von Stammzellen zu erzeugen, ist die Verwendung von Matrigel, welches aus Mäusetumoren gewonnen wird. Es handelt sich dabei um ein Gemisch von über 1800 Proteinen und Wachstumsfaktoren, von denen ein Großteil unbekannt ist und deren natürliche Organisation durch die Extraktion zerstört wird. Es adsorbiert an der Oberfläche mit einer unbekannten Struktur. Neben den offensichtlichen ethischen Problemen mit der gezielten Züchtung von Tumoren in Labortieren hat Matrigel den Nachteil, dass es nicht für biomedizinische Anwendungen verwendet werden kann, da die Gewebe Pathogene oder genetische Mutationen enthalten können und daher eine potentielle Gefahr darstellen.

Laminin-Beschichtungen können auch dadurch erzeugt werden, dass die Moleküle an das zu beschichtende Material chemisch angebunden werden. Hierzu muss das Material chemisch aktiviert werden, was seine Oberflächeneigenschaften verändert. Dazu werden reaktive Linker angebracht, die dann im nächsten Schritt mit Laminin reagieren. Die nicht umgesetzten Linker müssen danach noch deaktiviert werden. Dieses Verfahren erfordert eine Vielzahl von Schritten und bietet keine Kontrolle darüber, in welcher Orientierung die Laminin-Moleküle angebunden werden. Eine biomimetische Beschichtung wird so nicht erzeugt.

Die bekannten Verfahren weisen folglich Nachteile auf. Wie oben dargestellt, bildet das Laminin in einigen bekannten Verfahren kein geordnetes Netzwerk, da es sich nicht gemäß seiner räumlichen Struktur orientiert; es wird also keine bio-mimetische Schicht erzielt. In einigen Verfahren ist die Laminin-Schichtdicke nicht steuerbar und variiert in Abhängigkeit einer Vielzahl von zu erforschenden Parametern. Andere Verfahren sind ineffizient, weil sich nur ein geringer Teil des eingesetzten Laminins tatsächlich zu einer Schicht verbindet. Ein Großteil des eingesetzten Laminins muss dann verworfen werden. Weitere Verfahren sind zeitaufwändig und teuer, da sie eine Vielzahl von Verfahrensschritten erfordern. Schließlich sind einige Verfahren nicht für biomedizinische Anwendungen verwendbar, da sie Laminin und Kollagen verwenden, das aus humanem oder tierischem Gewebe extrahiert wurde. Diese Gewebe können Pathogene oder genetische Mutationen enthalten und stellen daher eine potentielle Gefahr dar.

Die WO 2010/019625 A2 offenbart orientierte Biopolymer- und Biokompositmaterialien auf Kollagenbasis, die als Träger für Stammzellen geeignet sind, sowie Verfahren zu deren Herstellung. Die DE 11 2019 002 479 B4 offenbart ein Verfahren zur Herstellung einer Kollagen-Laminin-Matrix zur Heilung von Hautgeschwüren, Verbrennungen und Wunden.

Trotz erheblicher Anstrengungen bei der Entwicklung von orientierten, kollagenbasierten Biokompositmaterialien wie synthetische Basallamina gibt es bis heute keine industrielle Herstellungsmethode für diese Materialien in Serienproduktion. Die vorliegende Erfindung hat sich die Aufgabe gestellt, ein Verfahren zur Herstellung von auf geordnetem Kollagen und Laminin basierten Biokompositmaterialien als synthetische Basallamina bereitzustellen, das die oben aufgeführten Nachteile nicht aufweist. Insbesondere sollen mit dem erfindungsgemäßen Verfahren einfach und effizient orientierte kollagenbasierte Biokompositmaterialien herstellbar sein, die für biomedizinische Anwendungen verwendbar sind und eine biomimietische Beschichtung aufweisen.

### ZUSAMMENFASSUNG DER ERFINDUNG

Gemäß einem Aspekt betrifft die vorliegende Erfindung ein Verfahren zur Herstellung von auf Kollagen und Laminin basierten Biokompositmaterialien, insbesondere synthetischen Basallamina gemäß Patentanspruch 1. Weitere Ausführungsformen sind in den Unteransprüchen beschrieben. Das erfindungsgemäße Verfahren führt zu neuartigen zweidimensionalen Biokompositmaterialien aus Kollagen und Laminin, vorzugsweise aus Kollagen IV und Laminin-111, mit hoher Packungsdichte und optimaler Anordnung des Laminins, bei denen sowohl Kollagen als auch Laminin in einer Monoschicht vorliegen. Folglich betrifft die Erfindung gemäß einem anderen Aspekt auch zweidimensionale Biokompositmaterialien aus Kollagen und Laminin, die gemäß dem beschriebenen Verfahren herstellbar sind.

Bei dem erfindungsgemäßen Verfahren wird zunächst eine wässrige, Pufferlösung, die auf einen pH-Wert von 3 bis 5, vorzugsweise 3,5 bis 4,5, wie etwa 4 eingestellt ist, bereitgestellt. Die Pufferlösung kann ein Alkalimetallsalz und/oder ein Erdalkalimetallsalz, beispielsweise ein Alkalimetallhalogenid wie Natrium- oder Kaliumchlorid und/oder ein Erdalkalimetllhalogenid wie Calciumchlorid enthalten. Auf die Subphase an der Luft-Wasser-Grenzfläche der Pufferlösung wird eine saure Kollagenlösung eingebracht, vorzugsweise durch Auftropfen. Anschließend wird die mit Kollagenlösung versetzte Pufferlösung für eine ausreichende Zeit ruhen gelassen, bis sich mindestens ein Teil des Kollagens zu einer Kollagenschicht an einer Luft-Wasser-Grenzfläche der Kollagenlösung vernetzt hat. Dann wird eine wässrige Laminin-Lösung in die Subphase unterhalb der Kollagenschicht an der Luft-Wasser-Grenzfläche eingebracht und für eine ausreichende Zeit inkubieren gelassen, bis sich eine Laminin-Beschichtung unterhalb der Kollagenschicht gebildet hat. Das an der Luft-Wasser-Grenzfläche der Lösung gebildete Biokomposit wird schließlich auf ein festes Substrat transferiert.

Es wurde überraschenderweise gefunden, dass das Laminin mit Hilfe des beschriebenen Verfahrens an der Kollagenschicht adsorbiert, mithin diese als die natürliche Umgebung "erkennt" und sich so anordnet (selbst organisiert), dass die langen Arme der Laminin-Moleküle von der Kollagenschicht weg zeigen. Es wurde überraschend gefunden, dass die so erzeugten Schichten in ihrer Organisation sehr viel mehr dem natürlichen Vorbild ähneln, als durch Adsorption aus Lösung oder Matrigel hergestellte Schichten, dass die Adhäsion von Zellen an die geordneten Lamininmoleküle stärker ist, als bei ungeordneten Lamininschichten vergleichbarer Dicke, dass der Bedarf an Kollagen und Laminin minimiert ist, da der Großteil des eingesetzten Materials auch übertragen wird und die Dicke und Organisation der Beschichtung wohldefiniert und unabhängig vom zu beschichtenden Material ist.

Gemäß einem Aspekt der Erfindung wird als Kollagen Kollagen IV verwendet, obwohl die Erfindung auch auf jegliche andere Netzwerk bildende Kollagene wie beispielsweise Kollagen VIII und Kollagen X verwendet werden können. Gemäß einem weiteren Aspekt wird als Laminin Laminin-111 verwendet, obwohl die Erfindung auch auf jegliche andere Laminine anwendbar ist. Es können rekombinante Laminine und Kollagene verwendet werden, was Vorteile gegenüber Proteinen hat, die aus humanem oder tierischem Gewebe extrahiert werden.

Die Laminin-Packdichte, bestimmt mittels Ellipsometrie, kalibriert für Kollagen IV, beträgt vorzugsweise 250 ng·cm⁻² bis 350 ng·cm⁻². Der Wert für die Laminin-Packdichte kann aus der Kollagen Kollagen IV Kalibration abgeleitet werden, weil beide alpha-helikale Proteine sind, die ähnliche optische Eigenschaften aufweisen.

### DETAILLIERTE BESCHREIBUNG DER ERFINDUNG

Es wurde gefunden, dass sich Kollagen und Laminin in einer Monoschicht an der Luft-Wasser-Grenzfläche, im Fall von Laminin bevorzugt unter sauren Bedingungen, selbst vernetzen. Je höher die Kollagen oder LamininKonzentration, desto mehr wird die Selbstvernetzung gefördert.

Nach dem Ausbreiten sowohl der Kollagen als auch Laminin Moleküle auf der wässrigen Lösung zeigte die Brewster-Winkelmikroskopie (BAM) eine glatte, auf der Mikroskala homogene Schicht, in der eine Zunahme der Reflektivität mit der Zeit deutlich erkennbar war.

Die Orientierung und die Verankerung des Laminins mit dem Kollagen wird ebenfalls durch das saure Medium gefördert. Gemäß einem Aspekt der Erfindung wird die Kollagenlösung als saure Lösung mit einem pH-Wert von 3 bis 5, vorzugsweise 3,5 bis 4,5, wie etwa 4 eingesetzt. Gemäß einem weiteren Aspekt wird als saure Kollagenlösung eine essigsaure Kollagenlösung verwendet.

Gemäß einem weiteren Aspekt der vorliegenden Erfindung wird die Kollagenlösung mit einer Kollagenkonzentration von 0,1 mg/mL bis 10 mg/mL, bevorzugt 0,5 bis 5 mg/mL, wie etwa 1 mg/mL bereitgestellt und auf in die Subphase der Luft-Wasser-Grenzfläche der Pufferlösung eingebracht wird. Das Einbringen erfolgt vorzugsweise durch Auftropfen, beispielsweise mit einer Pipette.

Darüber hinaus können die Ionen Na⁺, Cl⁻ und Ca²⁺, die im extrazellulären Raum von Zellen vorhanden sind, die intermolekularen Wechselwirkungen ebenfalls verändern. Einwertige Ionen wie Na⁺ und Cl⁻ unterstützen den starken Zusammenbau von Proteinen an der Grenzfläche, indem sie den hydrophoben Effekt fördern und die elektrostatischen Wechselwirkungen zwischen den Molekülen abschirmen. Zweiwertige Ca²⁺-Ionen sind vorteilhaft für die Polymerisation von Laminin in Bulklösung und auf Lipiden und aktivieren die Polymerisation durch Bindung an die gamma-Kurzketten von Laminin. Gemäß einem weiteren Aspekt der Erfindung umfasst die Pufferlösung weiterhin Alkalimetall- und/oder Erdalkalimetallsalze, bevorzugt Alkalimetall- und/oder Erdalkalimetallhalogenide. Bevorzugt liegen die Alkalimetallsalze in einer Konzentration von 50 mM bis 250 mM, bevorzugter 100 mM bis 200 mM, wie etwa 140 mM in der Kollagenlösung vor. Gemäß einem weiteren Aspekt der Erfindung liegen die Erdalkalimetallsalze in einer Konzentration von 1 mM bis 100 mM, bevorzugt 10 mM bis 50 mM in der Kollagen bzw. Lamininlösung vor. Die Alkalimetallsalze und oder die Erdalkalimetallsalze können - jedes für sich - entweder in der anfänglichen Pufferlösung vorgelegt oder zusammen mit der sauren Kollagenlösung in die Subphase der Pufferlösung eingebracht werden. Bevorzugt ist das Alkalimatallsalz ausgewählt aus Natriumchlorid und Kaliumchlorid, besonders bevorzugt Natriumchlorid. Das Erdalkalimetallsalz ist bevorzugt Calciumchlorid.

Gemäß einem weiteren Aspekt der vorliegenden Erfindung beträgt die Zeitspanne zur Vernetzung mindestens eines Teils des Kollagens zu einer Kollagenschicht an der Luft-Wasser-Grenzfläche der Pufferlösung 30 Minuten bis 12 Stunden, beispielsweise 1 Stunde bis 6 Stunden, wie etwa 2 Stunden.

Gemäß einem noch weiteren Aspekt der vorliegenden Erfindung wird die Lamininlösung mit einer Lamininkonzentration von 0,1 mg/mL bis 10 mg/mL, bevorzugt 0,5 bis 5 mg/mL, wie etwa 1 mg/mL bereitgestellt und in die Subphase der mit Kollagen versetzten Pufferlösung eingebracht.

Gemäß einem noch weiteren Aspekt der vorliegenden Erfindung beträgt die Zeitspanne zur Inkubation der Laminin-Lösung, bis sich eine Laminin-Beschichtung unterhalb der Kollagenschicht gebildet hat, 2 Stunden bis 24 Stunden, beispielsweise 6 Stunden bis 18 Stunden, wie etwa 12 Stunden bis 14 Stunden.

Der Transfer der gebildeten der bio-mimetischen Basallamina auf ein festes Substrat (Trägermaterial) parallel oder senkrecht zur Luft-Wasser-Grenzfläche kann mittels Langmuir-Schäfer-Transfer oder Langmuir-Blodgett-Transfer stattfinden. Beide Techniken sind aus dem Stand der Technik bereits bekannt.

Die erfindungsgemäß hergestellten Substrate finden z.B. Anwendung in der Wissenschaft und in der Diagnostik. Ferner können die Substrate zur Züchtung von Stammzellen verwendet werden. Die erfindungsgemäßen Laminin-Kollagen Doppelschichten sind weiterhin verwendbar für die Biologisierung von Implantaten, da sie die Integration in das Gewebe fördern. Die Methode eignet sich insbesondere für glatte Oberflächen wie z.B. in Elektroden, Schrauben, Fäden, Drähten. Die erfindungsgemäß hergestellten Laminin-Kollagen Doppelschichten können darüber hinaus definierte und naturnahe Epithelschichten aufbauen werden, welche sich für das pharmakologische Screening eignen, z.B. im Hinblick auf Wirkstoffpermeation und Pharmakokinetik.

### BEISPIEL

Für die Herstellung von Laminin/Kollagen-IV Doppelschichten wurden zunächst 100 pL von 1 mg/mL-1 Kollagen-IV in Essigsäure auf 5 mL Subphase aufgetragen und für 2 Stunden auf der Subphase (pH-Wert = 4 mit c = 100 mM NaCl) adsorbiert. Danach wurden mittels einer Spritze 50 µL von 1 mg/mL Laminin in die Subphase injiziert. Die Schichtbildung erfolgte über etwa 14 Stunden. Die Kollagen-IV-Lösung wurde vor dem Einspritzen der Laminin-111-Lösung nicht entfernt oder ausgetauscht. Für den Transfer auf ein festes Substrat wurde die Schicht einfach durch Berührung mit einem Substrat, das parallel zur Grenzfläche ausgerichtet war, nach der Langmuir-Schäfer-Transfer- Methode abgezogen. Fluoreszenzmikroskopie belegt, dass es sich um 2 separate Schichten handelt, da die Fluoreszenz von Laminin und Kollagen nicht kolokalisiert.

Um nachzuweisen, dass die Menge des abgelagerten Proteins bei jedem Transfer konstant ist, wurden Stapel von 1, 3 und 5 Schichten auf Si-Wafern und Goldbeschichteten Glasträgern hergestellt und mittels PM-IRRAS, Ellipsometrie und AFM charakterisiert. Die mit AFM ermittelte Dicke eines Lam-111 2D-Netzwerks war (3,2:1:1) nm. Die netzwerkartige Morphologie des Lam-111 2D-Netzwerks auf dem Si-Wafer ist im AFM-Phasenbild deutlich zu erkennen, während das Höhenbild auf eine einheitliche dünne Schicht (alle Merkmale unter 5 nm Höhe). Bei 5 Schichten betrug die Dicke das vierfache einer einzelnen Schicht bei (14,8:1:2,1) nm. Die Dicke wurde durch ellipsometrische Messungen bestätigt, bei denen die Dicke der Multischichten auf (8,9:1:0,1) nm für 3 Schichten und (16,1:1:0,3) nm für 5 Schichten. Die Dicke der Laminin-111-Monoschichten konnte ohne Kenntnis des Brechungsindexes der Schicht nicht direkt ellipsometrisch bestimmt werden, da die Dicke unterhalb des ultradünnen film-Grenze liegt. Die Packungsdichte der hergestellten Basallamina, bestimmt mittels Ellipsometrie betrug nach etwa 2 Stunden 315 ng·cm⁻² oder 3,7·10⁻¹³ mol·cm⁻². Die genaue Kontrolle über die flächenmäßige Proteinkonzentration wurde durch PM-IRRAS von Laminin-111-filmen auf goldbeschichtetem Glas, die nach jedem Transfer in einem mehrschichtigen Ablagerungsexperiment durchgeführt. Es zeigte sich ein schrittweisen linearen Anstieg der Proteinmenge (integrierte Amid I- und Amid II-Intensität) mit jedem Ablagerungsschritt.

## Patentansprüche

1. Verfahren zur Herstellung von Biokompositmaterialien aus Kollagen und Laminin umfassend die Schritte:
Bereitstellen einer wässrigen Pufferlösung, die auf einen pH-Wert von 3 bis 5 eingestellt ist;
Einbringen einer sauren Kollagenlösung in die Subphase an der Luft-Wasser-Grenzfläche der Pufferlösung
Ruhen lassen der mit saurer Kollagenlösung versetzten Pufferlösung für eine ausreichende Zeit, bis sich mindestens ein Teil des Kollagens zu einer Kollagenschicht an einer Luft-Wasser-Grenzfläche der Kollagenlösung vernetzt hat
Einbringen einer wässrigen Laminin-Lösung in die Subphase unterhalb der Kollagenschicht an der Luft-Wasser-Grenzfläche und Ruhen lassen der mit Laminin versetzten Pufferlösung für eine ausreichende Zeit, bis sich eine Laminin-Beschichtung unterhalb der Kollagenschicht gebildet hat
Transferieren des an der Luft-Wasser-Grenzfläche der Lösung gebildeten Biokomposits auf ein festes Substrat.

2. Verfahren nach Anspruch 1, bei dem als saure Kollagenlösung eine Kollagen-IV, Kollagen-VIII, Kollagen-X-Lösung oder eine Mischung derselben verwendet wird.

3. Verfahren nach Anspruch 1 oder Anspruch 2, bei dem als Laminin-Lösung eine Laminin-111-Lösung verwendet wird.

4. Verfahren nach einem der vorgehenden Ansprüche, bei dem die saure Kollagenlösung in die Subphase an der Luft-Wasser-Grenzfläche der Pufferlösung durch auftropfen eingebracht wird.

5. Verfahren nach einem der vorgehenden Ansprüche, bei dem das Ruhen lassen der mit saurer Kollagenlösung versetzten Pufferlösung in einer Zeitspanne von 30 Minuten bis 12 Stunden, vorzugsweise 1 Stunde bis 6 Stunden, besonders bevorzugt etwa 2 Stunden erfolgt.

6. Verfahren nach einem der vorgehenden Ansprüche, bei dem die wässrige Laminin-Lösung in die Subphase unterhalb der Kollagenschicht an der Luft-Wasser-Grenzfläche mittels einer Spritze eingebracht wird.

7. Verfahren nach einem der vorgehenden Ansprüche, bei dem das Ruhen lassen der mit Laminin versetzten Pufferlösung in einer Zeitspanne von 2 Stunden bis 24 Stunden, vorzugsweise 6 Stunden bis 18 Stunden, besonders bevorzugt 12 Stunden bis 14 Stunden erfolgt.

8. Verfahren nach einem der vorgehenden Ansprüche, bei dem rekombinantes Kollagen und rekombinantes Laminin verwendet wird.

9. Verfahren nach einem der vorgehenden Ansprüche, bei dem die Pufferlösung weiterhin Alkalimetall- und/oder Erdalkalimetallsalze, bevorzugt Alkalimetall- und/oder Erdalkalimetallhalogenide umfasst.

10. Verfahren nach Anspruch 9, bei dem als Alkalimetallhalogenid Natriumchlorid und/oder Kaliumchlorid verwendet wird.

11. Verfahren nach Anspruch 9 oder 10, bei dem als Erdalkalimetallhalogenid Calciumchlorid verwendet wird.

12. Verfahren nach einem der vorgehenden Ansprüche, bei dem die Kollagenlösung mit einer Kollagenkonzentration von 0,1 mg/mL bis 10 mg/mL, bevorzugt 0,5 bis 5 mg/mL bereitgestellt wird.

13. Verfahren nach einem der vorgehenden Ansprüche, bei dem die Lamininlösung mit einer Lamininkonzentration von 0,1 mg/mL bis 10 mg/mL, bevorzugt 0,5 bis 5 mg/mL bereitgestellt wird.

14. Biokompositmaterial aus Kollagen und Laminin herstellbar durch ein Verfahren gemäß einem der vorgehenden Ansprüche.

15. Biokompositmaterial nach Anspruch 14 mit einer Protein-Packdichte von 250 ng·cm⁻² bis 350 ng·cm⁻², bevorzugt 300 ng·cm⁻² bis 325 ng·cm⁻² bestimmt mittels Ellipsometrie, kalibriert für Kollagen IV.
